# EUROPEAN PATENT SPECIFICATION

(11) **EP 0 478 631 B1**
(45) Date of publication and mention of the grant of the patent: **24.08.1994**
(21) Application number: 90909466.6
(22) Date of filing: 22.06.1990
(51) Int. Cl.: A61K 43/00, A61K 47/48, A61K 49/02, A61K 37/32

(54) **TARGETTING AGENTS**
ZUM WIRKORT HINGEFÜHRTE UND DORT ANGEREICHERTE AKTIVSUBSTANZEN
AGENTS DE CIBLAGE

(30) Priority: 23.06.1989 GB 8914527; 03.04.1990 GB 9007469
(43) Date of publication of application: 08.04.1992
(73) Proprietor: CANCER RESEARCH CAMPAIGN TECHNOLOGY LTD., London NW1 4JL (GB)
(72) Inventor: PAGE-THOMAS, David Peter, Cambridge CR1 4RU (GB); BARD, David Roy, Cambridge CR1 4RU (GB); KNIGHT, Clive Graham, Cambridge CR1 4RU (GB)
(74) Representative: Molac, Béatrice
(86) International application number: GB9000966
(87) International publication number: WO9100111

(56) References cited:
- Journal of Medicinal Chemistry, vol. 32. no. 1, January 1989, the American Chemical Society, (Washington, DC, US), F. Al-Obeidi et al. pages 174-179
- Biochemical Society Transactions London, vol. 14, part 3, June 1986, The Biochemical Society, (London, GB), D.R. Bard et al. pages 614-615
- Journal of the American Chemical Society, vol. 111, no. 9, 26 April 1989, the American Chemical Society, (Washington, DC, US), F. Al-Obeidi et al. pages 3413-3416
- Neural and Endocrine Peptides and Recetors,(Annu. Wash. Spring Symp. Health Sci.), 1986, Plenum Press, (New York, US), M.E. Hadley et al.: pages 45-56

## Description

The present invention relates to targetted agents directed against malignant melanoma cells and to their production and use.

Malignant melanoma occurs at an annual rate of between 1 and 6 cases per 100,000 population worldwide. It is particularly prevalent in the caucasian populations of Australasia, the USA, Switzerland and Scandinavia and has shown a marked increase in incidence over the last two decades. Prognosis is poor in comparison with other dermal carcinomas due to the tendency of melanomas to early metastasis and their resistance to gamma irradiation and systemic cytotoxic chemotherapy. The success of therapy would be markedly improved by an agent which could target diagnostic or therapeutic agents to primary melanomas and their metastases.

Much work in this area has concentrated on the development of monoclonal antibodies to specific cell surface markers. Such an approach suffers from the difficulty of finding a cell surface marker unique to melanomas, but shared by all variants of the tumour and the recognition and elimination of the antibody molecule by the immune system of the patient.

An alternative approach utilises the observation that both malignant and normal melanocytes possess receptors for the peptide hormone alpha-melanocyte stimulating hormone (MSH). MSH stimulates the production of melanin by melanocytes in a wide range of chordate species. In mammals, it does so by combining with cell surface receptors, thus initiating a sequence of events which ultimately results in the stimulation of the synthesis of tyrosinase, the enzyme which converts tyrosine to L-DOPA, the immediate precursor of melanin.

MSH may be modified at the N-terminus with little or no loss in hormonal activity and MSH linked to diphtheria toxin in this manner has been shown selectively to kill melanoma cells in vitro. (Murphy, J. R. et al., Proc. Natl. Acad. Sci. USA, 83, 8258-8262 (1986).

So-called "super-potent" synthetic analogues of MSH have been described in the literature, for instance [Nle⁴, D-Phe⁷]-α-MSH (Sawyer T. K. et al., Proc. Natl. Acad. Sci. USA 77 5754-5788 (1980). A further super-potent analogue is [Nle⁴, Asp⁵, D-Phe⁷, Lys¹⁰] α-MSH₄₋₁₀.

MSH substituted with the chelator, diethylenetriamine pentaacetic acid (DTPA) at the N-terminus (MSH-DTPA), becomes rapidly associated with melanoma cells both in vitro and in vivo (Bard, D.R. et al., Biochem. Soc. Trans. London 14 614-615 (1986)).

It has now surprisingly been discovered that new targetted agents which comprise [Nle⁴, Asp⁵, D-Phe⁷, Lys¹⁰] alpha-MSH₄₋₁₀-NH₂ have improved binding to melanocytes and especially rapid clearance of unbound agent from animals to which the agents have been administered. The extremely rapid clearance of such targetted agents from the blood permits the use of short-lived but more active radio-isotopes as the targetted moiety for treating melanoma because the radiation dose is delivered more specifically to the melanoma and, indeed, the radiation dose to the patient as a whole may then be reduced.

Accordingly the present invention provides a product of formula (I)

R-[Nle⁴, Asp⁵, D-Phe⁷, Lys¹⁰] α-MSH₄₋₁₀ -NH₂. (I)

wherein R is a targetted moiety optionally bonded via a linking group. In formula (I) the group "-NH₂" simply indicates that the C-Terminus of the α-MSH₄₋₁₀ analogue polypeptide is amidated; such C-terminal amidation is important for activity.

The group R may be a directly bonded targetted moiety having at least one position to which the N-terminus of the α-MSH₄₋₁₀ analogue polypeptide may be attached without destroying the utility of the targetted moiety. Alternatively, the group R may be a bonded via a linking group which is itself bonded to the α-MSH₄₋₁₀ analogue.

Suitable targetted moieties include cytotoxic agents such as dacarbazine (5-[3,3-dimethyl-1-triazenyl]-1H-imidazole-4-carboxamide), actinomycin D, methotrexate and methyl CCNU (N-(2-chloroethyl)-N'-(trans-4-methylcyclohexyl)N-nitrosourea) and their precursors and cytokine agents such as tumor necrosis factor (TNF) and gamma interferon.

Other targetted moieties are detectable labels, such as radiolabels, for localisation and imaging of tumours. Particularly suitable radionuclides for use as cytotoxic agents include short half-life beta emitters such as erbium 169, yttrium 90, rhenium 186 and lutetium 177 and for use as imaging agents include indium 111, indium 113m and gallium 67 and especially indium 113m.

In general, small targetted moieties such as radionuclides are unlikely to be suitable for direct bonding to the α-MSH₄₋₁₀ analogue and will, therefore, be bonded via a linking group. Suitable linking groups for binding many types of targetted moiety to peptides will be known to those skilled in the art. For linking radionuclides it is particularly preferred to use chelating agents such as diethylenetriamine pentaacetic acid (DTPA); ethylenediamine tetraacetic acid (EDTA); ethylene glycol-O,O'-bis(2-aminoethyl)-N,N,N',N'-tetraacetic acid (EGTA); triethylenetetraminehexaacetic acid (TTHA) (Subramanian, K.M et al, J. Nucl. Med. 31(4) 480-488 (1990)); N,N'-bis(hydroxybenzyl)ethylenediamine-N,N'-diacetic acid (HBED); N,N',N'',N'''-tetraacetic acid derivatives of tetraazacycloalkanes such as 1,4,7,10-tetraazacyclododecane-N,N',N'',N'''-tetraacetic acid (Deshpande, S.V et al. J. Nucl, Med, 31($), 473-479 (1990), this is an ideal chelator for yttrium but the formation of complexes by this type of rigid ring structure is highly sensitive to the ionic radius of the ligand); 1,4,8,11-tetraazacyclotetradecane - N,N',N'',N'''-tetraacetic acid and 1,4,8,12-tetraazacyclopentadecane -N,N',N'',N'''-tetraacetic acid. In another aspect the invention provides a product of formula (II)

L-[Nle⁴, Asp⁵, D-Phe⁷, Lys¹⁰] α-MSH₄₋₁₀-NH₂ (II)

wherein L is a linking group, preferably a chelating agent such as DTPA.

A particular preferred compound of formula (II) is the compound of formula
DTPA-Nle-Asp-His-D-Phe-Arg-Trp-Lys-NH₂
Products of the present invention may be made by conventional techniques. In order to link the peptide to the targetted moiety or linking group, it will often be convenient to have the peptide bound to a solid substrate for instance the resin beads conventionally used in peptide synthesis. Where the targetted moiety or linking group is capable of binding two or more peptide chains it is preferred to use an excess of the targetted moiety or linking group and an acylation catalyst such as 1-hydroxybenzotriazole in order to favour formation of the singly substituted product. When the linking group is DTPA, the reaction may be conducted using a stoichiometric amount of DTPA bisanhydride but preferably an excess of DTPA bisanhydride is used. It is also possible to conduct the reaction using a large excess of DTPA bisanhydride.

The invention therefore further provides a process for producing a product of formula (I) comprising reacting a group R or a reactive derivative thereof with the α-MSH₄₋₁₀ analogue or a reactive derivative thereof. Where the group R is a targetted moiety bonded via a linking group the process will generally involve the steps of (a) reacting a group L or a reactive derivative thereof with α-MSH₄₋₁₀ analogue or reactive derivative thereof to form a product of formula (II) as hereinbefore defined and (b) reacting the product of formula (II), or a reactive derivative thereof with a compound R' or a reactive derivative thereof containing the targetted moiety R.

The reaction conditions such as temperature, duration and selection of solvents and the nature of the reactive derivatives will be determined by the nature of the groups R, L and R' and it is within the ability of the skilled person to select these as appropriate.

Preferably the process further involves separation and purification of the product. For products where the targetted moiety R' is bonded via a linking group L which is a chelating agent such as DTPA, the production process preferably involves synthesis of the peptide chains on a resin substrate by sequential addition of protected amino acid residues, N-terminal deprotection is necessary at this stage and is followed by reacting the peptide chains with the linking reagent. Thereafter the full deprotection of the amino acid residue side chains and removal of the peptide chains from the resin substrate are effected in either order. Finally, the agent so produced is purified, for instance by reverse phase chromatography.

Products according to the present invention are useful in the diagnosis and surgical or therapeutic treatment of melanoma. The present invention therefore further provides a product of formula (I) or (II) for use in a method of treatment of the human or animal body by surgery or therapy or in a method of diagnosis practised on the human or animal body. Also provided are diagnostic methods using the products of formula (I) or (II) in testing samples of tissue removed from the human or animal body. The invention further provides a method for the treatment or diagnosis of melanoma comprising administering an effective, non-toxic amount of a product of formula (I) or (II) to a human or animal having, or suspected to have a melanoma.

The products of formula (I) or (II) may be used directly or they may be administered in the form of a pharmaceutical composition comprising the product of formula (I) or (II) and a pharmaceutically acceptable diluent or carrier. Such diluents and carriers include tabletting agents, wetting agents, binders and fillers, preservatives such as antioxidants and antimicrobial agents, buffers and salts as well as conventional injection media, particularly water for injection. Such pharmaceutical compositions, and processes for formulating them, form further aspects of the present invention as does the use of a product of formula (I) or (I) in the preparation of a medicament for use in the diagnosis or surgical or therapeutic treatment of melanoma.

The products of formula (I) or (II), or compositions thereof, are administered by any conventional route including, enteral and parenteral routes especially by injection by the intravenous, intramuscular, subcutaneous or peritoneal routes.

Dosages and dosage regimes will depend on whether the agent is being administered for localisation or imaging purposes or as a therapeutic agent and on the age, health, weight and sex of the recipient. Typical dosages are likely to be in the region of from 0.001mg/kg to 0.5 mg/kg, for instance 0.003 mg/kg to 0.2mg/kg per day or even 0.02 or preferably 0.03 mg/kg to 0.2 mg/kg per day. For adult humans a normal daily dose is envisaged of from 0.05 mg to 50 mg, preferably from 0.01 mg to 10mg, for instance 0.5 mg to 5 mg. This dose may be administered as a single dose or as divided doses at intervals. Dosages would be repeated as necessary.

The invention will now be illustrated by the following Figures of the accompanying drawings and by the following Examples.

In the accompanying drawings:
Fig 1 shows a comparison between the activity of MSH, Ac-[Nle⁴, Asp⁵, D-Phe⁷, Lys¹⁰] α-MSH₄₋₁₀-NH₂ and a compound of the invention, DTPA-[Nle⁴, Asp⁵, D-Phe⁷, Lys¹⁰] α-MSH₄₋₁₀ -NH₂ in a tyrosinase assay.
Figs, 2 and 3 show the tissue distribution in mice after 4h
   (Fig. 2) and 24h (Fig. 3) following injection of 2.5 x 10⁻¹⁰ moles of DTPA-[Nle⁴, Asp⁵, D-Phe⁷, Lys¹⁰] α-MSH₄₋₁₀-NH₂ (hatched bars) or 2MSH-DTPA (open bars) each complexed with 3.6 microcuries (0.13Mbq) of In^{III}. 2MSH-DTPA is the compound:
   Ser-Tyr-Ser-Met-Glu-His-Phe-Arg-Trp-Gly-Lys-Pro-Val-NH₂
   DTPA
   Ser-Tyr-Ser-Met-Glu-His-Phe-Arg-Trp-Gly-Lys-Pro-Val-NH₂
Fig 4 shows the elimination of radioactivity from the circulation of mice following the injection of 2.5 x 10⁻¹⁰ moles of DTPA-[Nle⁴, Asp⁵, D-Phe⁷, Lys¹⁰] -MSH(₄₋₁₀)-NH₂ complexed with 3.6 microcuries (0.13 MBq) of ¹¹¹In.

### EXAMPLE 1

### Preparation of In¹¹¹ Labelled [Nle⁴, Asp⁵, D-Phe⁷,Lys¹⁰] alpha-MSH₄₋₁₀

### Synthesis of DTPA-(Nle⁴, Asp⁵, D-Phe⁷, Lys¹⁰) MSH₄₋₁₀

The peptide was synthesised step-wise from the C-terminus by the Fmoc-polyamide method using standard procedures on a Cambridge Research Biochemicals Pepsynthesiser (cf. Dryland, A. & Sheppard, R. C. Tetrahedron 44, 859-876 (1988)). Pepsyn KB resin (1 g, 0.2 mmoles of norleucine/g) was used and the linkage agent was Fmoc-amino-(2,4-dimethoxy-4'-carboxymethoxy)diphenylmethane. The Fmoc group of the linkage reagent was removed and the resulting amino-resin was treated with Fmoc-lysine(Boc)pentafluorophenyl ester and 1-hydroxybenzotriazole (0.6 mmoles of each). After 1 hour, coupling to the amine was complete and the resin was washed prior to removal of the Fmoc group. The next residue, tryptophan, was also added as the pentafluorophenyl ester. The third and fourth residues, arginine and D-phenylalanine, were coupled as free acids in the presence of benzotriazol-1-yloxy-tris(dimethylamino)phosphonium hexafluorophosphate (BOP) and di-isopropylethylamine (0.6 mmoles of each reagent). The remaining residues were added as the pentafluorophenyl esters. Side chains were protected as tert-butyl derivatives, except for arginine, for which the 2,2,5,7,8-pentamethylchroman-6-sulphonyl group was used.

When the assembly of the peptide was complete, the Fmoc group was removed from the N-terminal residue and the peptide resin was reacted with DTPA bis-anhydride (214 mg, 0.6 mmoles) in the presence of 1-hydroxybenzotriazole and di-isopropylethylamine (0.6 mmoles of each). Reaction was complete after 1.5 hrs and the resin was washed with dimethylformamide (DMF), water, DMF, acetic acid and ether. After drying, the resin (1.32 g) was gently stirred with a mixture of trifluoroacetic acid (58 ml), anisole (1 ml), ethanedithiol (0.5 ml) and indole (1 g.) After 2 hrs, the resin was filtered, washed with TFA and the combined filtrates rotary evaporated. Ether (100 ml) was added to the residue to precipitate the crude peptide (192 mg). This was purified by ion-exchange chromatography on a column (15 mm X 350 mm) of Fractogel TSK CM-650(M), eluted with 0.01M-ammonium acetate, pH 4.5. Fractions containing the peptide were combined and freeze-dried, and the product (53 mg) was further purified by reverse phase chromatography on a column (15 mm x 350 mm) of Vydac C18 (15 - 20 um) in a low pressure system. A linear gradient was applied from 5% (v/v) to 50% (v/v) acetonitrile in 0.1% TFA. The main peptide peak was freeze-dried to give 35 mg of material homogeneous on high pressure liquid chromatography. The identity of the product was confirmed by amino acid analysis and fast-atom-bombardment mass spectrometry which gave a relative molecular mass of 1374. The compound was labelled with In¹¹¹ by the method described in Comparative Example 1 below.

### EXAMPLE 2

### Synthesis of DTPA-(Nle⁴, Asp⁵, D-Phe⁷, Dab¹⁰) MSH₄₋₁₀.

This was made and purified exactly as described above, except that the first amino acid was L-diaminobutyric acid (Dab). The compound was labelled with In₁₁₁ by the method described in Comparative Example 1 below.

### COMPARATIVE EXAMPLE 1

### A) SYNTHESIS OF 2MSH-DTPA

The peptide was synthesised step-wise from the C-terminus by the Fmoc-polyamide method using standard procedures on a Cambridge Research Biochemicals Pepsynthesiser (cf. Dryland, A. & Sheppard, R. C. Tetrahedron 44 859-876 (1988)). Pepsyn K resin (2g, 0.1 mmoles of norleucine/g) was used and the linkage agent was Fmoc-amino-(2,4-dimethoxy-4'-carboxymethoxy)diphenylmethane. The Fmoc group of the linkage agent was removed and the resulting amino-resin was treated with Fmoc-valine pentafluorophenyl ester and 1-hydroxybenzotriazole (0.8 mmoles of each). After 30 min, coupling to the amine was complete and the resin was washed prior to removal of the Fmoc group and the coupling of the next residue. This procedure was repeated at each step using Fmoc amino acid pentafluorophenylesters, except in the case of serine which was coupled as the oxobenzotriazine ester. Side chains were protected as tert-butyl derivatives, except for arginine where the 4-methoxy-2,3,6-trimethyl-benzenesulphonyl group was used.

When the assembly of the peptide was complete, the Fmoc group was removed from the N-terminal residue and the peptide-resin was reacted with DTPA bis-anhydride (108 mg, 0.3 mmoles) in the presence of diisopropylethylamine (0.025 mL, 0.15 mmoles). Reaction was complete after 1 hr and the resin was washed with dimethylformamide (DMF), water, DMF, dichloromethane and ether. After drying, a portion of the resin (0.7 g) was treated overnight with a mixture of trifluoroacetic acid (TFA), ethane-dithiol, phenol and anisole (97:1:1:1, by vol).

Evaporation of the TFA solution followed by trituration with ether yielded a white solid (110mg). This was purified by reverse phase chromatography on a column (15 mm x 500 mm) of Vydac C18 (15 -20 um) in a low pressure system. A linear gradient was applied from 0.1% TFA in water to 35% (v/v) acetonitrile in 0.1% TFA over 1.5 L. Fractions containing 2MSH-DTPA were identified by high pressure liquid chromatography and combined and lyophilised to yield 11 mg. The identity of the product was confirmed by fast atom bombardment mass spectrometry which gave a relative molecular mass of 3602 ± 2.

### B PREPARATION OF ¹¹¹ INDIUM LABELLED 2MSH-DTPA

Peptide (0.5 mg) was dissolved in water (0.5ml) and citrate buffer (0.1M pH 5.6, 1.0ml) was added. This solution was labelled with ¹¹¹InCl₃ (1.0 MCi in 0.1 ml 0.04M hydrochloride acid (Amersham)). Binding to the peptide was confirmed by thin-layer chromatography on silica gel using a double ascent system. The first solvent was 10% aqueous ammonium acetate/methanol (1:1, v/v) and the second, methanol/water (8.5:1.5, v/v). In this system unbound indium forms a colloidal hydroxide which has an Rf of 0.5, whilst labelled peptide moves at the solvent front. 100% of the radioactivity was located at the solvent front when the solution was analysed immediately after the addition of indium, and after standing for 5h at 18°C.

### BIOLOGICAL DATA:

### A) Tyrosinase Activity In Vitro

Cloudman S91 were grown in monolayers to a density of approximately 25,000 cells cm⁻² in 25cm² culture flasks using 4 ml Ham's F10 medium supplemented with 10% heat inactivated foetal calf serum. The medium was replaced and 10⁻⁵ and 10⁻⁹ molar concentrations of the test or control compound added. After 24 h, medium and melantropins were renewed and 1»Ci, 3,5-³H-tyrosine included. The final specific activity of 3,5-³H-tyrosine incubation medium was 0.1 Ci/mmol.

After a further 24h, 0.2ml of the medium from each incubation were transferred to centrifuge tubes containing 1ml activated charcoal suspension (100 mg/ml in 0.1M citric acid). The tubes were vortexed and centrifuged at 700 x g for 5 min. Aliquots (0.5 ml) of each supernatant were transferred to scintillation vials and counted on a Packard Tri-Carb 300D scintillation counter. Aliquots (0.1ml) of the unextracted medium were also counted. Finally, the remaining medium was removed and the cell monolayer suspended in 1ml isotonic trypsin/EDTA. The dispersed cells were counted in a Coulter counter.

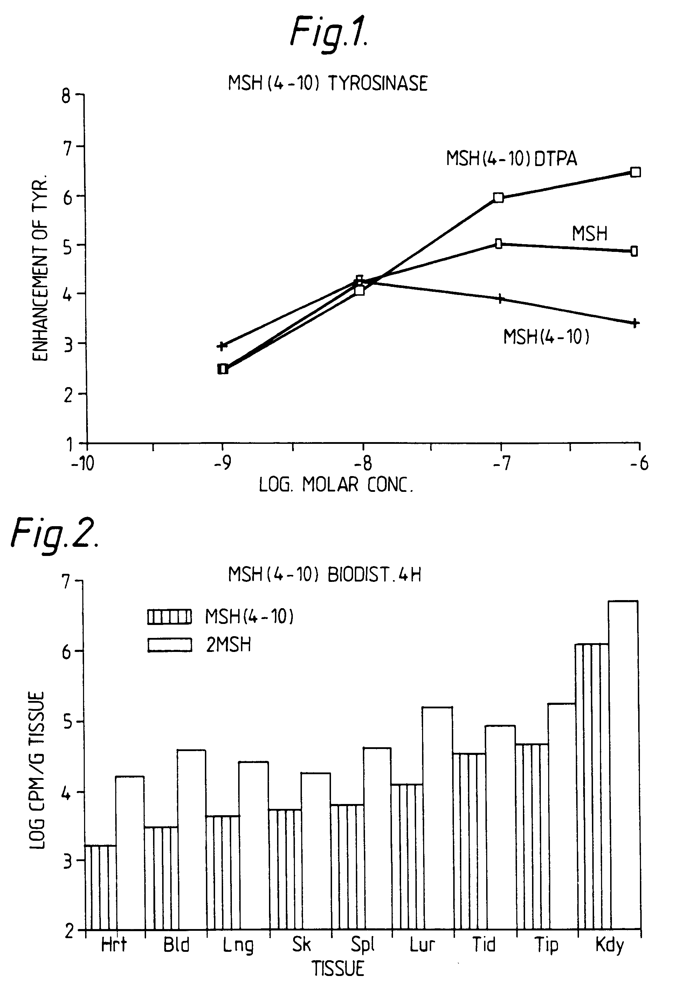

The amount of water generated from the tyrosine per 10⁵ cells was calculated and the results divided by the value obtained from the melanotropin-free control. Results are shown in Fig 1. The activities of the melanotropins were expressed as the molar concentrations required to give responses equal to half the maximum (EC₅₀) and are recorded in Table 1.

**TABLE 1**

| COMPOUND | EC₅₀ (nM) |
|---|---|
| MSH | 2.81 |
| MSH₍₄₋₁₀₎ | 0.74 |
| MSH₍₄₋₁₀₎ DTPA | 2.09 |

### B Comparison Testing in Vivo

2MSH-DPTA and the compound of formula (IIa) were labelled with ¹¹¹In by the general method described in Comparison Example 1(B) but using 2.5 nmol DTPA-peptide in 0.05 ml water, 0.2 ml citrate buffer and 5»Ci ¹¹¹In. The solution was adjusted to 2.5ml after the addition of indium with physiological saline. 0.1ml of this solution was injected i.p. into 20 DBA2 mice which had been inoculated with Cloudman 891 cells i.p. 14 days previously. In some experiments, mice were also injected with a solution prepared as above, but with the omission of DTPA-peptide.

Animals were killed in groups of 5 at 0.5,1,2,4 and 24h after injection of the labelled peptide. Blood (0.1-0.3 g), brain (whole), eyes, liver (approx. 1g), spleen (whole), heart (whole), lungs (whole), skeletal muscle (approx. 0.1g) and tumour (0.1-0.5g) induced intradermally (Tid) or intraperitoneally (Tip) were removed. The individual tissues were weighed and counted on a Packard Multi-Prias 4 gamma scintillation counter using a window of 50-500 KeV. Results were expressed as CPM/g wet weight and divided by the blood value at each time point.

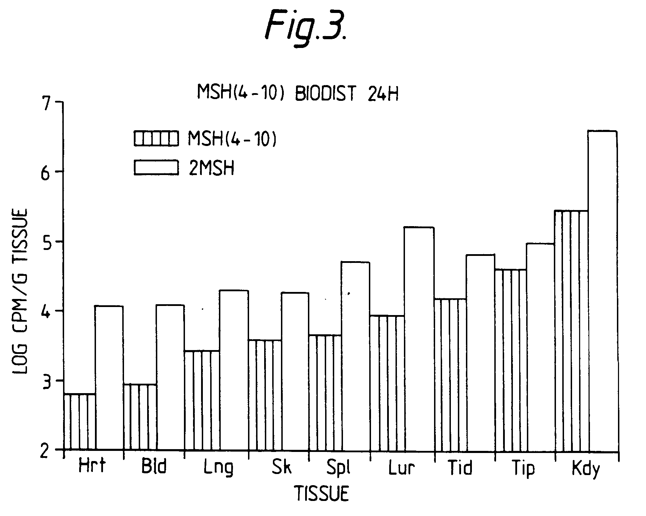

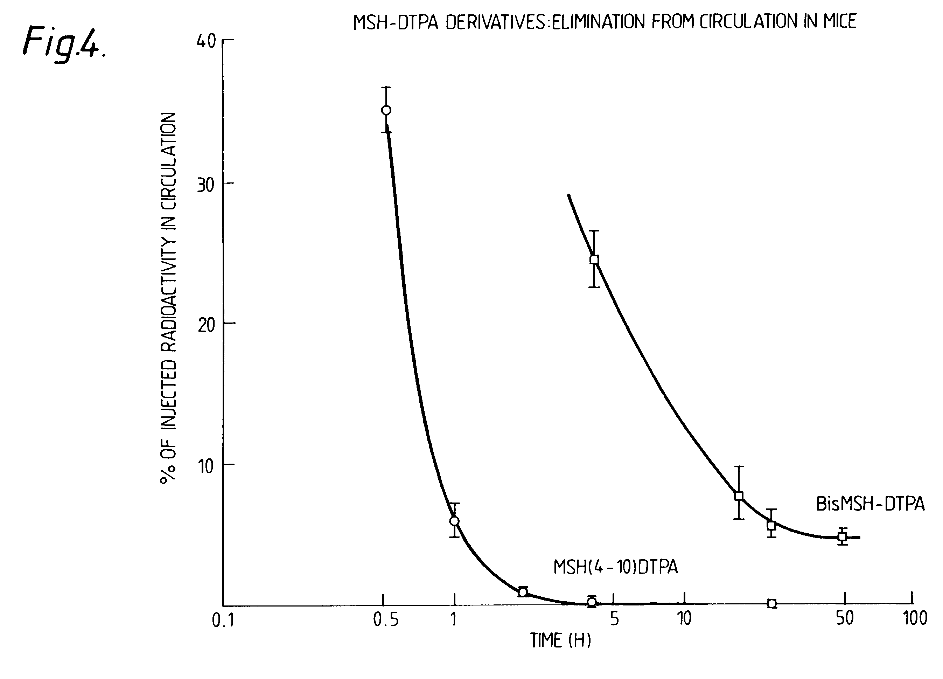

Results are shown in Figs. 2 to 3. The elimination of radio-activity from the circulation is shown in Fig 4.

## Claims (Claims for the following Contracting State(s): AT, BE, CH, DE, DK, FR, GB, IT, LI, LU, NL, SE)

1. A compound of formula
R - M
wherein R is a targetted moiety bonded, optionally via a linking group, to the N-terminal of an α-MSH₄₋₁₀ analogue polypeptide represented by M and having the formula
[Nle⁴, Asp⁵, D-Phe⁷, Lys¹⁰] α-MSH₄₋₁₀-NH₂.

2. A compound according to claim 1 wherein R is derived from 5 -(3,3 dimethyl-1-triazenyl)-1H-imidazole-4-carboxamide, actinomycin D, methotrexate, N-(2-chloroethyl)-N'-(trans-4-methylcyclohexyl)-N-nitrosourea or precursors thereof, tumour necrosis factor, gamma interferon; or is a radionuclide selected from erbium 169, yttrium 90, rhenium 186, lutetium 177, indium 111, indium 113m and gallium 67.

3. A compound according to claim 1 or claim 2 wherein the moiety R is a radionuclide bonded to the group M via a linking group derived from diethylenetriamine pentaacetic acid, ethylenediamine tetraacetic acid, ethylene glycol-O,O'-bis-(2-aminoethyl)-N,N,N',N'-tetraacetic acid, triethylenetetramine hexaacetic acid or N,N'-bis(hydroxybenzyl)-ethylenediamine-N,N'-diacetic acid.

4. A compound of formula
L - M
wherein L is a linking group and M is as defined in claim 1.

5. A compound according to claim 4 wherein L is derived from diethylenetriamine pentaacetic acid, ethylenediamine tetraacetic acid, ethylene glycol-O,O'-bis-(2-aminoethyl)-N,N,N',N'-tetraacetic acid, triethylenetetramine hexaacetic acid or N,N'-bis(hydroxybenzyl)-ethylenediamine-N,N'-diacetic acid.

6. The compound of formula
DTPA-Nle-Asp-His-D-Phe-Arg-Trp-Lys-NH₂

7. The compound of formula
DTPA-(Nle⁴,Asp⁵,D-Phe⁷,Dab¹⁰)MSH₄₋₁₀

8. A process for producing a compound according to any one of claims 1 to 3 comprising reacting a moiety R or a reactive derivative thereof with an α-MSH₄₋₁₀ analogue polypeptides of formula
[Nle₄, Asp₅, D-Phe⁷, Lys¹⁰] α-MSH₄₋₁₀ -NH₂
or a reactive derivative thereof.

9. A process according to claim 8 wherein the reactive derivative of the α-MSH₄₋₁₀ analogue polypeptide is a compound of formula according to claim 4.

10. A process for producing a compound according to claim 4 or claim 5 comprising reacting a moiety L or a reactive derivative thereof with an α-MSH₄-₁₀ analogue polypeptide of formula
[Nle⁴, Asp⁵, D-Phe⁷, Lys¹⁰) α-MSH₄₋₁₀-NH₂
or a reactive derivative thereof.

11. A compound according to any one of claims 1 to 7 for use in a method of diagnosis or treatment practised on the human or animal body.

12. Use of a compound according to any one of claims 1 to 7 in the manufacture of a medicament for use in a method of diagnosis or treatment of melanoma practised on the human or animal body.

13. A pharmaceutical composition comprising a compound according to any one of claims 1 to 7 and a pharmaceutically acceptable carrier or diluent therefor.

## Claims (Claims for the following Contracting State(s): ES)

1. A process for producing a compound of formula
R - M
wherein R is a targetted moiety bonded, optionally via a linking group, to the N-terminal of an α-MSH₄₋₁₀ analogue polypeptide represented by M and having the formula [Nle⁴, Asp⁵, D-Phe⁷, Lys¹⁰] α-MSH₄₋₁₀-NH₂, which process comprises reacting a moiety R or a reactive derivative thereof with an α-MSH₄₋₁₀ analogue polypeptide of formula
[Nle₄, Asp₅, D-Phe⁷, Lys¹⁰] α-MSH₄₋₁₀-NH₂
or a reactive derivative thereof.

2. A process according to claim 1 wherein R is derived from 5-(3,3 dimethyl-1-triazenyl)-1H-imidazole-4-carboxamide, actinomycin D, methotrexate, N-(2-chloroethyl)-N'-(trans-4-methylcyclohexyl)-N-nitrosourea or precursors thereof, tumour necrosis factor, gamma interferon; or is a radionuclide selected from erbium 169, yttrium 90, rhenium 186, lutetium 177, indium 111, indium 113m and gallium 67.

3. A process according to claim 1 or claim 2 wherein the moiety R is a radionuclide bonded to the group M via a linking group derived from diethylenetriamine pentaacetic acid, ethylenediamine tetraacetic acid, ethylene glycol-O-O'-bis-(2-aminoethyl)-N,N,N',N'tetraacetic acid, triethylenetetramine hexaacetic acid or N,N'-bis(hydroxybenzyl)-ethylenediamine-N,N'-diacetic acid.

4. A process according to claim 1 wherein the reactive derivative of the α-MSH₄₋₁₀ analogue polypeptide is a compound of formula
L - M
wherein L is a linking group and M is as defined in claim 1.

5. A process according to claim 4 wherein L is derived from diethylenetriamine pentaacetic acid, ethylenediamine tetraacetic acid, ethylene glycol-O,O'-bis-(2-aminoethyl)-N,N,N',N'-tetraacetic acid, triethylenetetramine hexaacetic acid or N,N'-bis(hydroxybenzyl)-ethylenediamine-N,N'-diacetic acid.

6. A process for producing a compound of formula
L - M
wherein L is a linking group and M is as defined in claim 1, comprising reacting a moiety L or a reactive derivative thereof with an α-MSH₄₋₁₀ analogue polypeptide of formula
[Nle⁴, Asp⁵, D-Phe⁷, Lys¹⁰] α-MSH₄₋₁₀-NH₂
or a reactive derivative thereof.

7. A process according to claim 6 wherein the compound prepared is of formula
DTPA-Nle-Asp-His-D-Phe-Arg-Trp-Lys-NH₂

8. A process according to claim 6 wherein the compound prepared is DTPA-(Nle⁴, Asp⁵, D-Phe⁷, Dab¹⁰)MSH₄₋₁₀.

## Patentansprüche (Patentansprüche für folgende(n) Vertragsstaat(en): AT, BE, CH, DE, DK, FR, GB, IT, LI, LU, NL, SE)

1. Eine Verbindung der Formel
R-M
worin R ein zielgerichteter Teil ist, der gegebenenfalls durch eine Verknüpfungsgruppe an den N-Terminus eines zu α-MSH₄₋₁₀ analogen Polypeptids dargestellt durch M und die Formel
[Nle⁴, Asp⁵, D-Phe⁷, Lys¹⁰] α-MSH₄₋₁₀-NH₂ hat, gebunden ist.

2. Eine Verbindung gemäss Anspruch 1 worin R von 5-(3,3-Dimethyl-1-triazenyl)-1H-imidazol-4-carboxamid, Actinomycin D, Methotrexat, N-(2-Chloroethyl)-N'-(trans-4-methylcyclohexyl)-N-nitrosoharnstoff oder Vorgängern davon, Tumor Necrosis Faktor, gamma Interferon hergeleitet oder ein Radionuklid ausgewählt aus Erbium 169, Yttrium 90, Rhenium 186, Lutetium 177, Indium 111, Indium 113m und Gallium 67 ist.

3. Eine Verbindung gemäss Anspruch 1 oder Anspruch 2 worin der Teil R ein Radionuklid ist, das an die Gruppe M mittels einer Verknüpfungsgruppe hergeleitet von Diethylentriaminpentaessigsäure, Ethylendiamintetraessigsäure, Ethylenglycol-O,O'-bis-(2-aminoethyl)-N,N,N',N'-tetraessigsäure, Triethylentetraminhexaessigsäure oder N,N'-Bis(hydroxybenzyl)-ethylendiamin-N,N'-diessigsäure gebunden ist.

4. Eine Verbindung der Formel
L-M
worin L eine Verknüpfungsgruppe und M wie in Anspruch 1 definiert ist.

5. Eine Verbindung gemäss Anspruch 4 worin L von Diethylentriaminpentaessigsäure, Ethylendiamintetraessigsäure, Ethylenglycol-O,O'-bis(2-aminoethyl)-N,N,N',N'-tetraessigsäure, Triethylentetraminhexaessigsäure oder N,N'-bis(hydroxybenzyl)-ethylendiamin-N,N'-diessigsäure hergeleitet ist.

6. Die Verbindung der Formel
DTPA-Nle-Asp-His-D-Phe-Arg-Trp-Lys-NH₂

7. Die Verbindung der Formel
DTPA-(Nle⁴ ,Asp⁵ ,D-Phe⁷ ,Dab¹⁰)MSH₄₋₁₀

8. Verfahren zur Herstellung einer Verbindung gemäss irgendeinem der Ansprüche 1 bis 3, das die Umsetzung eines Teils R oder eines reaktiven Derivates davon mit einem zu α-MSH₄₋₁₀ analogen Polypeptid der Formel
[Nle⁴ ,Asp⁵ ,D-Phe⁷ ,Lys¹⁰]α-MSH₄₋₁₀-NH₂ oder einem reaktiven Derivat davon umfasst.

9. Verfahren gemäss Anspruch 8 worin das reaktive Derivat des zu α-MSH₄₋₁₀ analogen Polypeptids eine Verbindung der Formel gemäss Anspruch 4 ist.

10. Verfahren zur Herstellung einer Verbindung gemäss Anspruch 4 oder Anspruch 5, das die Umsetzung eines Teils L oder eines reaktiven Derivates davon mit einem zu α-MSH₄₋₁₀ analogen Polypeptid der Formel
[Nle⁴ ,Asp⁵ ,D-Phe⁷ ,Lys¹⁰]α-MSH₄₋₁₀-NH₂ oder einem reaktiven Derivat davon umfasst.

11. Eine Verbindung gemäss irgendeinem der Ansprüche 1 bis 7 zur Verwendung in einer Diagnose- oder Behandlungsmethode, die auf menschlichem oder tierischem Körper angewendet wird.

12. Verwendung einer Verbindung gemäss irgendeinem der Ansprüche 1 bis 7 in der Herstellung eines Arzneimittels zur Verwendung in einer Diagnose- oder Behandlungsmethode von Melanoma, die auf menschlichem oder tierischem Körper angewendet wird.

13. Pharmazeutische Zusammensetzung enthaltend eine Verbindung gemäss irgendeinem der Ansprüche 1 bis 7 zusammen mit einem pharmazeutisch annehmbaren Trägerstoff oder Verdünnungsmittel.

## Patentansprüche (Patentansprüche für folgende(n) Vertragsstaat(en): ES)

1. Verfahren zur Herstellung einer Verbindung der Formel
R-M
worin R ein zielgerichteter Teil ist, der gegebenenfalls durch eine Verknüpfungsgruppe an den N-Terminus eines zu α-MSH₄₋₁₀ analogen Polypeptids dargestellt durch M und die Formel
[Nle⁴, Asp⁵, D-Phe⁷, Lys¹⁰] α-MSH₄₋₁₀-NH₂ hat, gebunden ist, wobei das Verfahren die Umsetzung eines Teils R oder eines reaktiven Derivates davon mit einem zu α-MSH₄₋₁₀ analogen Polypeptid der Formel
[Nle⁴ ,Asp⁵ ,D-Phe⁷ ,Lys¹⁰]α-MSH₄₋₁₀-NH₂ oder einem reaktiven Derivat davon umfasst.

2. Verfahren gemäss Anspruch 1 worin R von 5-(3,3-Dimethyl-1-triazenyl)-1H-imidazol-4-carboxamid, Actinomycin D, Methotrexat, N-(2-Chloroethyl)-N'-(trans-4-methylcyclohexyl)-N-nitrosoharnstoff oder Vorgängern davon, Tumor Necrosis Faktor, gamma Interferon hergeleitet oder ein Radionuklid ausgewählt aus Erbium 169, Yttrium 90, Rhenium 186, Lutetium 177, Indium 111, Indium 113m und Gallium 67 ist.

3. Verfahren gemäss Anspruch 1 oder Anspruch 2 worin der Teil R ein Radionuklid ist, das an die Gruppe M mittels einer Verknüpfungsgruppe hergeleitet von Diethylentriamin-pentaessigsäure, Ethylendiamintetraessigsäure, Ethylenglycol-O,O'-bis(2-aminoethyl)-N,N,N',N'-tetraessigsäure, Triethylentetraminhexaessigsäure oder N,N'-bis(hydroxybenzyl)-ethylendiamin-N,N'-diessigsäure gebunden ist.

4. Verfahren gemäss Anspruch 1 worin das reaktive Derivat des zu α-MSH₄₋₁₀ analogen Polypeptids eine Verbindung der Formel
L-M
worin L eine Verknüpfungsgruppe und M wie in Anspruch 1 definiert ist.

5. Verfahren gemäss Anspruch 4 worin L von Diethylentriaminpentaessigsäure, Ethylendiamintetraessigsäure, Ethylenglycol-O,O'-bis-(2-aminoethyl)-N,N,N',N'-tetraessigsäure, Triethylentetraminhexaessigsäure oder N,N'-bis(hydroxybenzyl)-ethylendiamin-N,N'-diessigsäure hergeleitet ist.

6. Verfahren zur Herstellung einer Verbindung der Formel
L-M
worin L eine Verknüpfungsgruppe und M wie in Anspruch 1 definiert ist, das die Umsetzung eines Teils L oder eines reaktiven Derivats davon mit einem zu α-MSH₄₋₁₀ analogen Polypeptid der Formel
[Nle⁴, Asp⁵, D-Phe⁷, Lys¹⁰] α-MSH₄₋₁₀-NH₂ hat, gebunden ist oder eines reaktiven Derivats davon umfasst.

7. Verfahren gemäss Anspruch 6 worin die hergestellte Verbindung die Formel
DTPA-Nle-Asp-His-D-Phe-Arg-Trp-Lys-NH₂
hat.

8. Verfahren gemäss Anspruch 6 worin die hergestellte Verbindung die Formel
DTPA-(Nle⁴ ,Asp⁵ ,D-Phe⁷ ,Dab¹⁰)MSH₄₋₁₀
hat.

## Revendications (Revendications pour l'(les) Etat(s) contractant(s) suivant(s): AT, BE, CH, DE, DK, FR, GB, IT, LI, LU, NL, SE)

1. Un composé de formule
R-M
dans laquelle R signifie un reste ciblant lié, éventuellement par l'intermédiaire d'un groupe de liaison, au N-terminal d'un polypeptide analogue de la α-MSH₄₋₁₀ représenté par M et répondant à la formule
[Nle⁴, Asp⁵, D-Phe⁷, Lys¹⁰] α-MSH₄₋₁₀-NH₂

2. Un composé selon la revendication 1, dans lequel R est dérivé du 5-(3,3-diméthyl-1-triazényl)-1H-imidazole-4-carboxamide, de l'actinomycine D, du méthotrexate, de la N-(2-chloroéthyl)-N'-(trans-4-méthylcyclohexyl)-N-nitroso-urée ou de leurs précurseurs, du facteur nécrosant les tumeurs, de l'interféron gamma, ou signifie un radionucléide choisi parmi erbium 169, yttrium 90, rhénium 186, lutétium 177, indium 111, indium 113m et gallium 67.

3. Un composé selon la revendication 1 ou 2, dans lequel le reste R est un radionucléide lié au groupe M par l'intermédiaire d'un groupe de liaison dérivé de l'acide diéthylènetriamine-pentaacétique, de l'acide éthylènediamine-tétraacétique, de l'acide éthylèneglycol-O,O'-bis-(2-aminoéthyl)-N,N,N',N'-tétraacétique, de l'acide triéthylènetétramine-hexaacétique ou de l'acide N,N'-bis(hydroxybenzyl)-éthylènediamine-N,N'-diacétique.

4. Un composé de formule
L-M
dans laquelle L signifie un groupe de liaison et M est tel que défini à la revendication 1.

5. Un composé selon la revendication 4, dans lequel L est dérivé de l'acide diéthylènetriamine-pentaacétique, de l'acide éthylènediamine tétraacétique, de l'acide éthylèneglycol-O,O'-bis-(2-aminoéthyl)-N,N,N',N'-tétraacétique, de l'acide triéthylènetétramine-hexaacétique ou de l'acide N,N'-bis-(hydroxybenzyl)-éthylènediamine-N,N'-diacétique.

6. Le composé de formule
DTPA-Nle-Asp-His-D-Phe-Arg-Trp-Lys-NH₂

7. Le composé de formule
DTPA-(Nle⁴, Asp⁵, D-Phe⁷, Dab¹⁰)MSH₄₋₁₀

8. Un procédé de préparation d'un composé selon l'une quelconque des revendications 1 à 3, comprenant la réaction d'un reste R ou l'un de ses dérivés réactifs avec un polypeptide analogue de la α-MSH₄₋₁₀ de formule
[Nle₄, Asp₅, D-Phe⁷, Lys¹⁰] α-MSH₄₋₁₀-NH₂
ou un dérivé réactif de ce composé.

9. Un procédé selon la revendication 8, dans lequel le dérivé réactif du polypeptide analogue de la α-MSH₄₋₁₀ est un composé répondant à la formule de la revendication 4.

10. Un procédé de préparation d'un composé selon la revendication 4 ou 5, comprenant la réaction d'un reste L ou l'un de ses dérivés réactifs avec un analogue de la α-MSH₄₋₁₀ de formule
[Nle⁴, Asp⁵, D-Phe⁷, Lys¹⁰] α-MSH₄₋₁₀-NH₂
ou un dérivé réactif de ce composé.

11. Un composé selon l'une quelconque des revendications 1 à 7, pour l' utilisation dans une méthode de diagnostic ou de traitement pratiquée sur le corps humain ou animal.

12. L'utilisation d'un composé selon l'une quelconque des revendications 1 à 7, pour la préparation d'un médicament destiné à l'utilisation dans une méthode de diagnostic ou de traitement des mélanomes pratiquée sur le corps humain ou animal.

13. Une composition pharmaceutique comprenant un composé selon l'une quelconque des revendications 1 à 7 et un véhicule ou diluant pharmaceutiquement acceptable.

## Revendications (Revendications pour l'(les) Etat(s) contractant(s) suivant(s): ES)

1. Un procédé de préparation d'un composé de formule
R-M
dans laquelle R signifie un reste ciblant lié, éventuellement par l'intermédiaire d'un groupe de liaison, au N-terminal d'un polypeptide analogue de la α-MSH₄₋₁₀ représenté par M et répondant à la formule [Nle⁴, Asp⁵, D-Phe⁷, Lys¹⁰] α-MSH₄₋₁₀-NH₂, lequel procédé comprend la réaction d'un reste R ou l'un de ses dérivés réactifs avec un polypeptide analogue de la α-MSH₄₋₁₀ de formule
[Nle₄, Asp₅, D-Phe⁷, Lys¹⁰] α-MSH₄₋₁₀-NH₂
ou un dérivé réactif de ce composé.

2. Un procédé selon la revendication 1, dans lequel R est dérivé du 5-(3,3-diméthyl-1-triazényl)-1H-imidazole-4-carboxamide, de l'actinomycine D, du méthotrexate, de la N-(2-chloroéthyl)-N'-(trans-4-méthylcyclohexyl)-N-nitroso-urée ou de leurs précurseurs, du facteur nécrosant les tumeurs, de l'interféron gamma, ou signifie un radionucléide choisi parmi erbium 169, yttrium 90, rhénium 186, lutétium 177, indium 111, indium 113m et gallium 67.

3. Un procédé selon la revendication 1 ou 2, dans lequel le reste R est un radionucléide lié au groupe M par l'intermédiaire d'un groupe de liaison dérivé de l'acide diéthylènetriamine-pentaacétique, de l'acide éthylènediamine-tétraacétique, de l'acide éthylèneglycol-O,O'-bis-(2-aminoéthyl)-N,N,N',N'-tétraacétique, de l'acide triéthylènetétramine-hexaacétique ou de l'acide N,N'-bis-(hydroxybenzyl)-éthylènediamine-N,N'-diacétique.

4. Un procédé selon la revendication 1, dans lequel le dérivé réactif du polypeptide analogue de la α-MSH₄₋₁₀ est un composé de formule
L-M
dans laquelle L signifie un groupe de liaison et M est tel que défini à la revendication 1.

5. Un procédé selon la revendication 4, dans lequel L est dérivé de l'acide diéthylènetriamine-pentaacétique, de l'acide éthylènediamine-tétraacétique, de l'acide éthylèneglycol-O,O'-bis-(2-aminoéthyl)-N,N,N',N'-tétraacétique, de l'acide triéthylènetétramine-hexaacétique ou de l'acide N,N'-bis-(hydroxybenzyl)-éthylènediamine-N,N'-diacétique.

6. Un procédé de préparation d'un composé de formule
L-M
dans laquelle L signifie un groupe de liaison et M est tel que défini à la revendication 1, comprenant la réaction d'un reste L ou l'un de ses dérivés réactifs avec un polypeptide analogue de la α-MSH₄₋₁₀ de formule
[Nle⁴, Asp⁵, D-Phe⁷, Lys¹⁰] α-MSH₄₋₁₀ -NH₂
ou un dérivé réactif de ce composé.

7. Un procédé selon la revendication 6, dans lequel le composé préparé répond à la formule
DTPA-Nle-Asp-His-D-Phe-Arg-Trp-Lys-NH₂.

8. Un procédé selon la revendication 6, dans lequel le composé préparé est le DTPA-(Nle⁴, Asp⁵, D-Phe⁷, Dab¹⁰)MSH₄₋₁₀.
